Europäisches Patentamt

European Patent Office

Office européen des brevets

(11) Veröffentlichungsnummer: **0 420 057 A1**

## (12) EUROPÄISCHE PATENTANMELDUNG

(21) Anmeldenummer: 90118161.0

(22) Anmeldetag: 21.09.90

(51) Int. Cl.5: **A61B 1/00**, G02B 23/26

(30) Priorität: 29.09.89 DE 3932515

(43) Veröffentlichungstag der Anmeldung:
03.04.91 Patentblatt 91/14

(84) Benannte Vertragsstaaten:
BE DE FR GB IT

(71) Anmelder: **Richard Wolf GmbH**
**Pforzheimer Strasse 32**
**W-7134 Knittlingen(DE)**

(72) Erfinder: **Diener, Jörg**
**Bremichstrasse 20**
**W-7519 Oberderdingen(DE)**

(74) Vertreter: **Wilcken, Thomas, Dipl.-Ing. et al**
**Musterbahn 1**
**W-2400 Lübeck(DE)**

(54) **Fokussierung von Endoskopobjektiven.**

(57) Bei der Vorrichtung zum Fokussieren eines Endoskopobjektivs (4) in Abhängigkeit vom Abstand des Objektes vom distalen Endoskopende ist das Objektiv (4) durch eine verdrehbare Schraubspindel (10) betätigbar. Die Spindel (10) greift in eine Mutter (11) des Objektivs (4) ein und verschiebt dieses durch ihre Verdrehung axial. Die Verdrehung der Schraubspindel (10) erfolgt über eine biegsame Welle (12), die proximal mit einer Hülse (13) verbunden und über eine von einer Handhabe (18) verdrehbaren Stange (15) einstellbar ist. Das distale in die Hülse eingreifende Ende der Spindel (10) ist axial in der Hülse (13) verschiebbar angeordnet und mit dieser Hülse verdrehbar verbunden.

Fig.1

EP 0 420 057 A1

Die Erfindung geht von einer Vorrichtung zum Fokussieren nach dem Oberbegriff des Anspruches 1 aus, wie es aus der DE-PS 1 253 407 und der DE-PS 1 269 287 bekannt ist.

In der einen bekannten Ausführung erfolgt die axiale Verstellung des Objektivs durch elektromagnetische Verschiebung des Objektivs von einer in eine andere Endstellung, so daß nur in diesen beiden Endstellungen eine Bildschärfe erreichbar ist (DE-PS 1 253 407).

In der zweiten bekannten Ausführung ist bei festliegendem Endoskopobjektiv das distale Ende des Bildleitfaserbündels verstellbar (DE-PS 1 269 287). Der Nachteil dieser Fokussierung besteht darin, daß die Bildschärfeneinstellung von dem verwendeten Material des Endoskop-Hüllschlauches und andererseits von der Krümmung eines flexiblen Endoskops abhängt, so daß ständige Nachfokussierung bei Bewegung des flexiblen Endoskopes erforderlich ist.

Die Aufgabe der Erfindung besteht darin, die Vorrichtung zum Fokussieren des Objektivs so auszubilden, daß innerhalb der Verstellweglänge in der zylindrischen Aufnahme ein Fokussieren des Objektivs möglich ist, die auch bei Bewegung des oder aus der Körperhöhle oder technischen Hohlraumes herausragenden Endoskopteiles erhalten bleiben soll. Diese Aufgabe wird durch die Merkmale des Anspruches 1 gelöst.

Vorteilhafte Ausgestaltungen sind durch die Unteransprüche zum Ausdruck gebracht.

Durch diese Lösung ist es möglich, die einmal eingestellte Fokussierstellung des Objektivs auch bei Bewegungen oder des aus der Körperhöhle oder aus einem technischen Hohlraum herausragenden Endoskopteiles beizubehalten, da sich die Länge der verdrehbaren Welle Längenänderungen bei solchen Bewegungen anpassen kann und der Gewindeeingriff der Schraubspindel in die Mutter des Objektivs die Beibehaltung jeder Objektivstellung gewährleistet.

Die Erfindung ist nachstehend anhand der Zeichnung erläutert. Es zeigen:

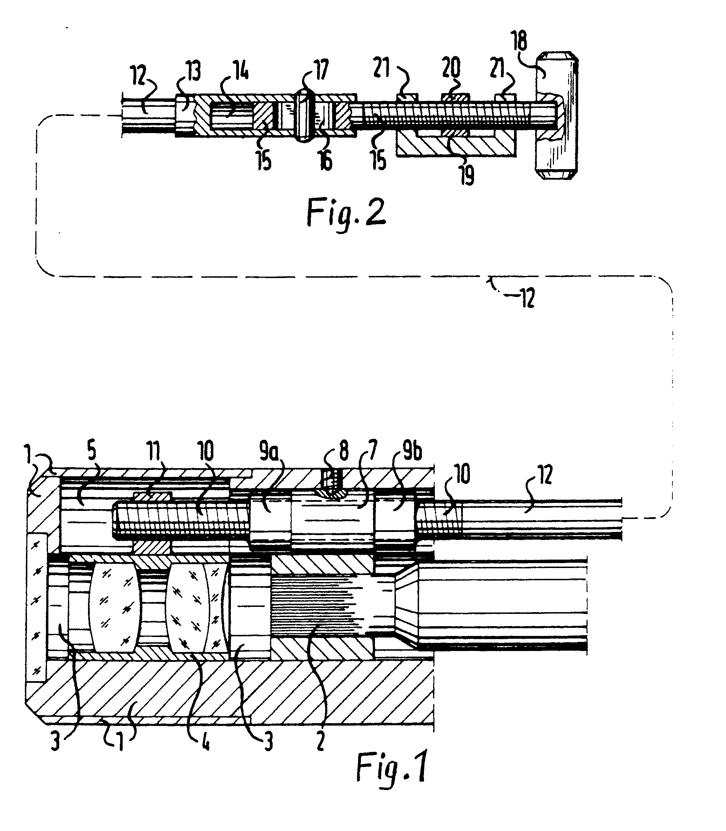

Figur 1 das distale Ende eines flexiblen Endoskops im Längsschnitt mit dem distalen Teil der Fokussierungsvorrichtung,

Figur 2 die proximale Vorrichtung zur Betätigung der Fokussierungseinstellung nach Figur 1.

Im distalen, starren Kopf 1 eines flexiblen Endoskopes ist vor dem distal festgelegten Ende eines Bildleitbündels 2 ein axial über eine begrenzte Länge des Zylinderraumes 3 verstellbares Objektiv 4 gelagert. Parallel zur optischen Achse des Objektivs ist in einem Längsschlitz 5 der Wandung des Kopfes 1 eine Schraubspindel 10 verdrehbar, aber axial unbewegbar gelagert. Hierzu ist im Anschluß an den Schlitz 5 ein Ringlager 7 vorgesehen, welches durch eine Schraube 8 oder sonstige Mittel lösbar festgelegt ist. Das Ringlager 7 wird von einer Schraubspindel 10 durchgriffen, auf der zwei Anschläge 9a und 9b befestigt sind, die gegen die Endflächen des Ringlagers 7 anliegen und dadurch die Schraubspirale 10 axial unbeweglich halten.

Das distale Ende der Schraubspindel 10 durchgreift eine Mutter 11, die mit sehr geringem Spiel in dem Längsschlitz 5 geführt und starr mit dem Objektiv 4 verbunden ist. An das proximale Ende der Schraubspindel 10 schließt sich eine biegsame, durch das Endoskop verlaufende Welle 12 an, die an ihrem proximalen Ende mit einer Hülse 13 fest verbunden ist. In den zylindrischen Innenraum 14 der Hülse 13 ist das distale Ende einer Stange 15 eingeschoben.

Die Stange 15 ist auf ihrem distalen, in der Hülse 13 liegenden Ende mit einem Längsschlitz 16 versehen, der von einem in der Hülsenwand festgelegten Zapfen 17 durchgriffen ist. Die Stange 15 ist am proximalen Ende mit einer Handhabe 18 versehen. Die Stange 15 mit der Handhabe 18 ist zwischen den Schenkeln 21 eines U-förmigen Lagers 19 dadurch axial begrenzt bewegbar, daß ein Anschlagelement 20 in den Einstellungen gegen die Anschläge 21 zur Anlage kommt.

Durch die vorbeschriebene Ausbildung kann durch Verdrehung der Handhabe 18 die Stange 15, die Hülse 13 und die Welle 12 zur Umdrehung mitgenommen werden, so daß sich damit auch die Schraubspindel 10 verdreht und damit die Mutter 11 und das Objektiv 4 axial verstellt, womit es möglich ist, die Bildschärfe in Abhängigkeit vom Abstand des Objekts vom Objektiv einzustellen, wobei die Einstellung durch den Gewindeeingriff der Teile 10 und 11 aufrechterhalten wird.

Durch die beschriebene Fokussiervorrichtung kann bspw. der proximale, aus einer Körperhöhle oder aus einem technischen Hohlraum herausragende Endoskopteil beliebig bewegt und gekrümmt werden, ohne daß sich dadurch die eingestellte Bildschärfenlage des Objektivs ändert, da sich die Verbindung zwischen dem distalen und proximalen Teil der Fokussiervorrichtung bei auftretenden Längenänderungen durch die Bewegung des Endoskopteiles mittels der möglichen axialen Verschiebung der Stange 15 in der Hülse 13 an diese Längenänderungen anpaßt.

## Ansprüche

1. Vorrichtung zum Fokussieren eines Objektivs eines starren oder eines flexiblen Endoskops durch axiales Verstellen des Objektivs in Abhängigkeit vom Abstand des Objektes vom Endoskopende, dadurch gekennzeichnet, daß das Objektiv (4) im starren distalen Kopf (1) des Endoskopes durch eine verdrehbar gelagerte Schraubspindel (10) über eine biegsame Welle (12) von einer proximalen, im Endoskop festliegenden Handhabe (18) mittels eines in einer und gegenüber einer proximalen Wellenhülse verdrehbaren, aber in der Hülse (13) axial frei verschiebbaren distalen Endes einer Stange (15) axial einstellbar ist.

2. Vorrichtung nach Anspruch 1, dadurch gekennzeichnet, daß mit der Fassung des Objektivs (4) eine in einem Längsschlitz (5) der starren distalen Endoskopwandung geführte Mutter (11) verbunden ist, mit der die axial unbewegliche Schraubspindel (10) in Eingriff steht.

3. Vorrichtung nach Anspruch 1 oder 2, dadurch gekennzeichnet, daß die Lagerung des distalen Endes der Schraubspindel aus einem Ringzylinder (7) besteht, der im distalen Endoskop ende fixiert ist und daß die Schraubspindel (10) mit zwei auf ihr festliegenden Anschlägen (9a,9b) versehen ist, die gegen die Enden des Zylinderringes (7) anliegen.

4. Vorrichtung nach einem der Ansprüche 1 bis 3, dadurch gekennzeichnet, daß die mit der Handhabe (18) verbundene Stange (15) mit ihrem distalen, einen Längsschlitz (16) aufweisenden Ende in der Wellenhülse (13) längsverschieblich gelagert ist und daß durch den Längsschlitz (16) ein in der Wandung der Wellenhülse (3) festgelegter Zapfen (17) verläuft.

5. Vorrichtung nach einem der Ansprüche 1 bis 4, dadurch gekennzeichnet, daß das Verstellen des Objektivs (1) zwischen seinen beiden Endstellungen durch ein auf der Stange (15) verstellbares Anschlagelement (20) und durch die beiden Schenkel (21) des U-förmigen Lagers (19) begrenzbar ist.

EP 0 420 057 A1

Fig.2

Fig.1

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-B-2 237 621 (OLYMPUS OPTICAL CO LTD)<br>* Spalte 4, Zeile 38 - Spalte 5, Zeile 45; Figuren 3A,B * | 1,2 | A 61 B 1/00<br>G 02 B 23/26 |
| A | EP-A-0 066 374 (OLYMPUS OPTICAL CO LTD)<br>* Zusammenfassung; Figuren 1,2 * | 1 | |
| A | DE-B-2 328 595 (K.K. MASCHIDA SEISAKUSHO)<br>* Spalte 2, Zeilen 24-50; Figuren 1,2 * | 1 | |

| | |
|---|---|
| | **RECHERCHIERTE SACHGEBIETE (Int. Cl.5)**<br><br>A 61 B 1/00<br>G 02 B 23/00 |

Der vorliegende Recherchenbericht wurde für alle Patentansprüche erstellt

| Recherchenort | Abschlußdatum der Recherche | Prüfer |
|---|---|---|
| BERLIN | 21-12-1990 | WEIHS J.A. |